# FASCICULE DE BREVET EUROPEEN

(11) **EP 3 222 256 B1**
(45) Date de publication et mention de la délivrance du brevet: **20.02.2019**
(21) Numéro de dépôt: 17162425.7
(22) Date de dépôt: 22.03.2017
(51) Int. Cl.: A61F 5/058

(54) **ATTELLE ENVELOPPANTE**
UMHÜLLENDE SCHIENE
ENVELOPING SPLINT

(30) Priorité: 24.03.2016 FR 1670132
(43) Date de publication de la demande: 27.09.2017
(73) Titulaire: VOGINNOV, 67000 Strasbourg (FR)
(72) Inventeur: VOGT, Jean-Claude, 67000 Strasbourg (FR)
(74) Mandataire: Barny, Luc

(56) Documents cités:
- EP-A1- 0 359 635
- EP-A2- 0 004 204
- FR-A3- 2 530 946
- US-A- 3 232 289
- US-A- 4 383 526

## Description

La présente invention concerne, de manière générale, une attelle, plus particulièrement en forme de « L », à usage unique permettant l'immobilisation par enveloppement d'un membre humain et plus particulièrement d'une jambe ou d'un bras.

Différents traumatismes, notamment osseux ou articulaires nécessitent l'immobilisation plus ou moins prolongée d'un membre blessé.

Ces traumatismes sont particulièrement fréquents.

L'immobilisation du membre permet, seule ou associée à d'autres soins, la consolidation osseuse et la cicatrisation tendineuse.

L'immobilisation est traditionnellement obtenue par la réalisation d'un plâtre enveloppant confectionné à l'aide de bandelettes enduites de plâtre enroulées autour du membre. Des bandes de résine polymérisant à l'air ou des plastiques thermoformables sont également utilisés de manière courante.

Les techniques d'immobilisation par plâtrage présentent différents inconvénients. Elles sont notamment d'une mise en oeuvre longue, fastidieuse et salissante. En outre elles produisent, tant en poids qu'en volume, un montant important de déchets, potentiellement contaminés. Ces inconvénients ont bien évidemment un coût.

Dans un système de soins cherchant à diminuer ses coûts il existe donc un réel besoin de dispositifs alternatifs.

Cependant malgré leurs nombreux inconvénients les plâtres restent largement utilisés faute de solutions alternatives présentant un résultat équivalent.

L'art antérieur contient un brevet US 2,700,383 qui propose une attelle de jambe réalisée à partir de matériaux peu coûteux tels que le plastique ou la fibre de bois compressée. Ces matériaux sont cependant encore lourds si on les compare à du carton ondulé, ce qui représente un inconfort certain pour le malade. En outre le dispositif proposé ne permet pas un enveloppement complet du membre. Enfin il n'est pas pliable.

L'art antérieur contient aussi un brevet US 3,624,745 pour l'immobilisation d'un membre inférieur consistant en une gouttière réalisée en carton ondulé maintenue par des bandes. Ce dispositif pliable n'est cependant que partiellement enveloppant et n'assure qu'une immobilisation temporaire. En outre, sa forme rectiligne ne permet pas l'immobilisation de la cheville et du pied.

L'art antérieur contient également un brevet US 3,496,934 qui procure un meilleur enveloppement du membre que le brevet précité mais qui, conservant une forme rectiligne, ne permet pas non plus l'immobilisation d'un membre présentant un angle.

L'art antérieur contient également le brevet russe 2 150 918 qui propose la réalisation d'une attelle obtenue à partir de carton ondulé. Cette attelle est composée de plusieurs profilés de section rectangulaires coulissant les uns dans les autres. Ce dispositif est enveloppant et procure une rigidité importante. Cependant celle-ci est obtenue par la collaboration des différents profilés coulissants, et donc nécessairement linéaires, et de renforts métalliques. L'utilisation de ces moyens limite l'utilisation de cette attelle à la seule partie non coudée d'un membre. Elle interdit en outre la réalisation de radiographies par rayons X.

Enfin l'art antérieur contient également un brevet EP 0004204 qui décrit une attelle enveloppante réalisée en feuille de plastique ondulé pliable pour réaliser un profilé formant un angle et apte à soutenir un membre. Ce dispositif léger ne prévoit cependant aucun moyen apte à assurer une rigidité satisfaisante du dispositif et éviter l'ouverture et/ou la fermeture dudit angle.

La présente invention a pour objet de pallier ce manque de l'art antérieur en proposant une attelle enveloppante permettant l'immobilisation d'un membre formant un angle, par exemple au niveau du coude ou de la cheville, dont la mise en place est aisée et rapide, dont la production et l'élimination sont peu onéreuses et qui assure une immobilisation équivalente à celle d'un plâtre.

Idéalement une telle attelle doit également pouvoir être stockée facilement et donc présenter un encombrement réduit.

Une fois mise en place, l'attelle présente sensiblement la forme d'un L.

Le chirurgien connaît l'angle d'immobilisation optimal. Par exemple pour un grand nombre d'indication la cheville sera immobilisée selon un angle à 90°. Cette même articulation pourra aussi être immobilisée à 110° pour une chirurgie du tendon d'Achille. Pour une entorse du coude l'angle d'immobilisation pourra être de 60°. Ces angles d'immobilisation sont en nombre très limité et il n'est donc pas besoin d'avoir une attelle à angle variable, un petit nombre d'attelles conçues avec des angles différents permettant de traiter tous les cas.

L'immobilisation du membre est assurée d'une part, par la rigidité de l'ensemble qui permet notamment un blocage des articulations, coude, poignet, genou et cheville et, d'autre part, par l'enveloppement du membre. L'enveloppement du membre exige du dispositif une certaine flexibilité. Pour réaliser une attelle fonctionnelle, la problématique de l'homme du métier est donc de conjuguer flexibilité et rigidité selon l'axe considéré.

C'est l'objet de la présente invention. Plus particulièrement, après de nombreux essais, la demanderesse a mis en évidence que des matériaux légers, très bon marché et recyclables tels que le carton pouvaient parfaitement être utilisés pour confectionner une attelle de membre en « L ». Une attelle selon l'invention peut parfaitement être conçue de manière à être pliable et donc stockable à plat dans un espace réduit puis dépliée - ou assemblée si elle est constituée de parties distinctes- en un temps très réduit et sans utilisation de locaux ou d'outils.

Dans un souci de clarté, et sans mention contraire dans le texte, les expressions suivantes devront être considérées comme ayant le sens défini.

Par « membre » on entend, sauf lorsqu'il en est précisé autrement le membre inférieur humain s'étendant du haut de la cuisse au bout du pied ou le membre supérieur humain s'étendant du haut du bras au bout de la main. Les références au membre dans l'espace correspondent au membre d'un sujet debout.

Par « partie supérieure du membre » on entend i) la partie, ou une portion substantielle de celle-ci, qui s'étend à partir du talon au haut de la cuisse quand on fait référence au membre inférieur et ii) la partie qui s'étend du coude au haut du bras ou une portion substantielle de celle-ci quand on fait référence au membre supérieur. Par partie « substantielle » on entend i) une partie qui s'étend du talon jusqu'à au moins la naissance du mollet quand on fait référence au membre inférieur ou ii)une partie qui s'étend du coude jusqu'à mi biceps quand on fait référence au membre supérieur.

Par « partie inférieure du membre» on entend i) le pied ou une portion substantielle de celui-ci quand on fait référence au membre inférieur et ii) la partie qui s'étend du coude au bout des doigts ou une portion substantielle de celle-ci quand on fait référence au membre supérieur. Par partie « substantielle » on entend une partie qui i) s'étend du talon jusqu'à au moins la naissance des doigts de pied quand on fait référence au membre inférieur ou ii)une partie qui s'étend du coude jusqu'au milieu de l'avant bras quand on fait référence au membre supérieur.

Par « articulation » on entend soit l'articulation de la cheville, soit l'articulation du coude.

Par « carton » on entend du papier, recyclé ou non, présenté sous une forme dont la masse est supérieure à 224g/mètre carré, réalisé à base de cellulose de fibre végétale ou tout autre composé d'origine végétale, présenté sous la forme de feuille simple ou de carton ondulé présentant une ou plusieurs épaisseurs de cannelures entre des feuilles de carton léger.

Par « matériau analogue » au carton on entend les matériaux non-métalliques pouvant être usinés sous forme de feuille et présentant des caractéristiques en termes de rapport masse/volume et de rigidité, similaires à celles du carton, par exemple des plastiques ou des matériaux obtenus à partir d'amidons.

Par « dispositif de verrouillage » on entend un dispositif destiné à fixer deux parties de l'attelle ensemble, pratiqué respectivement dans la même pièce que chacune de ces parties et faisant coopérer deux éléments désignés par convention « mâle » et « femelle ». Cela peut être par exemple une languette et une fente, la languette pouvant avantageusement être munie d'appendices dépliables de manière à ce qu'une fois dépliée la languette soit plus large que la fente dans laquelle elle vient s'insérer. Cela peut aussi être un cran coopérant avec une fente ou deux crans coopérant entre eux ou tout autre moyen connu de l'homme du métier. Un tel dispositif présente l'avantage d'être facile à mettre en oeuvre et de ne pas nécessiter l'utilisation de colle, agrafes ou autre moyen similaire.

Par « feuille » on entend une feuille simple ou un assemblage de feuilles de carton ou matériau équivalent présentant ou non une ou plusieurs épaisseurs de cannelure ou d'ondulation.

Par « ligne de pliage » on comprend un alignement de perforations, une incision linéaire continue ou discontinue de profondeur inférieure à l'épaisseur de la feuille, un pré-pliage ou tout autre moyen équivalent connu de l'homme du métier et permettant le pliage de la feuille selon deux plans s'étendant de part et d'autre de la ligne. En d'autres termes, il s'agit d'avoir une arête formant charnière.

Par « ouate » on entend toute bourre, par exemple de coton, de polyester, de viscose médicalement acceptable pour réaliser des pansements.

Selon une première forme d'exécution de la présente invention, il est proposé une attelle enveloppante réalisée en feuille de carton ou matériaux analogues et constituée d'un profilé d'un seul tenant formant un angle compris entre 135° et 45° et comprenant de part et d'autre de cet angle :
a. une partie (I) d'immobilisation de la partie inférieure du membre comprenant :
   i. une semelle,
   ii. au moins deux flancs positionnés symétriquement le long de la semelle pour former un canal capable de recevoir la partie inférieure du membre, et
   iii. au moins un rabat agencé le long de l'un des flancs et adapté pour fléchir longitudinalement pour fermer par recouvrement ledit canal et envelopper la partie inférieure du membre ; et
b. une partie (II) d'immobilisation de la partie supérieure du membre comprenant :
   i. au moins une tige postérieure,
   ii. au moins deux panneaux latéraux positionnés symétriquement le long de la tige postérieure pour former un canal capable de recevoir la partie supérieure du membre, et
   iii. au moins un panneau antérieur agencé le long de l'un des panneaux latéraux et adapté pour fléchir longitudinalement pour fermer par recouvrement ledit canal et envelopper la partie supérieure du membre,
ladite attelle étant caractérisée en ce que ledit profilé coudé est doublé par au moins une partie de renfort latérale formant équerre au niveau de ce coude au moins sur tout ou partie des flancs latéraux et des panneaux latéraux de manière à assurer la rigidité dudit coude.

Selon l'invention une première partie du canal (partie I) est destinée à accueillir la partie inférieure du membre alors que la seconde partie du canal (partie II) est destinée à accueillir la partie supérieure du membre.

Dans un mode préféré de réalisation de l'invention, les rabats sont au nombre de deux, disposés le long de chaque flanc. Pour permettre un meilleur enveloppement de la partie inférieure du membre, les rabats peuvent présenter une ou plusieurs lignes de pliage longitudinales.

Dans un autre mode de réalisation préféré de l'invention les panneaux antérieurs sont au nombre de deux, disposés respectivement le long de chaque panneau latéral. Pour faciliter l'enveloppement de la partie supérieure du membre, l'attelle selon l'invention peut également comprendre des panneaux intermédiaires agencés entre les panneaux latéraux et les panneaux antérieurs. Toujours avec le même objectif d'enveloppement de qualité, la tige postérieure peut présenter une ou plusieurs lignes de pliage longitudinales.

Bien entendu il ne s'agit que de modes de réalisation particuliers de l'invention parmi d'autres.

Réalisée en carton ou matériaux analogues, l'attelle selon l'invention a l'avantage d'être légère et de présenter la rigidité nécessaire pour assurer l'immobilisation du membre. Dans un mode de réalisation préférée de l'invention l'attelle est réalisée en carton ondulé double couche - c'est-à-dire deux couches d'ondulations prises entre trois feuilles - d'une épaisseur totale comprise entre de 2mm et 5mm. De manière encore plus préférentielle cette épaisseur est de 3mm.

Il existe toutefois des qualités et des densités de carton variable et leur rigidité n'est pas nécessairement proportionnelle à leur épaisseur. L'homme du métier saura en puisant dans ses connaissances générales adapter l'épaisseur du carton en fonction de sa qualité pour obtenir la rigidité souhaitée. Cette remarque vaut également pour les matériaux analogues.

Toujours selon l'invention le carton ou le matériau analogue utilisé pour réaliser l'invention peut être traité en surface ou doublé d'une feuille par exemple pour lui conférer une qualité hydrofuge, antiseptique ou hypoallergénique.

Le soignant pose l'attelle selon l'invention en y callant le membre du patient au moyen d'ouate et la referme simplement en pliant les rabats le long des lignes de pliage et en immobilisant l'ensemble par exemple en le sanglant au moyen de bandes collantes ou de bandes velcro® placées à intervalles réguliers. Le calage du membre peut bien entendu être obtenu par tout moyen connu de l'homme du métier tel que notamment le bandage du membre. Bien entendu l'agrafage des rabats à leur rabat en vis-à-vis est un autre moyen de fermeture de l'attelle.

Au besoin les rabats et panneaux antérieurs peuvent être retaillés par le soignant par exemple avec une paire de ciseaux pour adapter leur taille. Ils peuvent aussi être simplement positionnés en recouvrement de leur vis-à-vis. D'une manière générale tout ou partie de l'attelle selon l'invention peut être retaillée de manière très aisée pour l'ajuster aux dimensions du membre, ce qui n'exclut bien entendu pas de réaliser différentes tailles d'attelles pour éviter justement d'avoir à pratiquer un tel ajustement.

L'attelle selon l'invention peut être décorée par exemple de différents dessins ou décorable, ce qui peut permettre une meilleure acceptation de son port par exemple par un jeune patient. Bien entendu la présence de surfaces planes en carton permet toutes sortes d'impression. Par exemple celle de la taille de l'attelle, son mode d'emploi ou des consignes d'utilisation.

L'attelle selon l'invention peut être réalisée avec différents renforts, de tailles différentes et placés à des endroits différents. Le matériau choisi pour réaliser l'attelle selon l'invention permet de tels renforts. De tels renforts peuvent par exemple être aménagés en prolongeant à 90° les flancs et/ou les panneaux latéraux dans leur extrémité située du côté du coude du canal pour former des équerres assurant la rigidité de ce coude.

Dans un mode plus élaboré de l'invention, il est proposé un renfort important de la structure de l'attelle.
Toujours dans un mode préféré de réalisation de l'invention, le renfort double tout ou partie de la semelle, des flancs latéraux et des panneaux latéraux. Dans un autre mode préféré de réalisation de l'invention le renfort double tout ou partie de la tige postérieure, des flancs latéraux et des panneaux latéraux. Selon un mode de réalisation, l'attelle selon l'invention est caractérisée en ce que ledit doublage comprend au moins :
a) une partie de renfort inférieur choisie dans le groupe A comprenant :
   i) une partie de renfort inférieur intérieur (III) ou
   ii) une partie de renfort inférieur extérieur (III') et/ou;
b) une partie de renfort supérieur choisie dans le groupe B comprenant :
   i) une partie de renfort supérieur intérieur (IV) ou
   ii) une partie de renfort supérieur extérieur (IV').

Le renfort a pour fonction d'augmenter la rigidité des Parties I et II de l'attelle pour éviter tout mouvement ou contrainte (notamment en cisaillement ou en torsion) imposé à une des parties du membre.

Le renfort selon l'invention peut être indifféremment placé à l'intérieur de l'attelle ou à l'extérieur de cette dernière.

Dans ce dernier agencement, le carton ou le matériau analogue utilisé pour réaliser le renfort peut être d'une épaisseur plus importante dans la mesure où ce renfort ne vient pas en concurrence avec l'espace destiné à accueillir le membre. Bien entendu le renfort lui-même peut-être d'épaisseur variable et être réalisé à partir d'une ou plusieurs feuilles.

L'attelle renforcée selon l'invention peut avantageusement être pourvue d'un dispositif de verrouillage permettant de maintenir la partie de renfort inférieur intérieur (III) ou la partie de renfort inférieur extérieur (III') en place. Ainsi il est proposé selon l'invention une attelle qui est caractérisée en ce que la partie de renfort inférieur choisie entre la partie de renfort inférieur intérieur (III) et la partie de renfort inférieur extérieur (III') comporte au moins un moyen de verrouillage adapté pour coopérer avec un moyen de verrouillage positionné sensiblement sur la ligne de pliage entre la partie (I) et la partie (II) ou sur la partie (II).

L'attelle renforcée selon l'invention peut avantageusement être pourvue d'un dispositif de verrouillage permettant de maintenir la partie de renfort supérieur intérieur (IV) ou la partie de renfort supérieur extérieur (IV') en place.

Ainsi il est proposé selon l'invention une attelle qui est caractérisée en ce que la partie de renfort supérieur choisie entre la partie de renfort supérieur intérieur (IV) et la partie de renfort supérieur extérieur (IV') comporte au moins un moyen de verrouillage adapté pour coopérer avec un moyen de verrouillage positionné sensiblement sur la ligne de pliage entre la partie (I) et la partie (II) ou sur la partie (I).

Afin de faciliter les soins, l'attelle selon l'invention peut être proposée avec une garniture interne solidaire de l'attelle de façon à éviter qu'elle ne glisse.

Ainsi il est proposé selon l'invention une attelle qui est caractérisée en ce que ses faces internes accueillant tout ou partie du membre sont garnies d'une ouate de protection.

Un tel mode de réalisation permet de positionner une attelle complète avec un minimum de gestes et en un temps minimum. Ceci présente un avantage important en situation d'urgence.

Bien entendu une telle garniture pourra être complétée par le soignant, notamment en effectuant un bandage autour du membre.

Toujours dans l'esprit d'offrir un produit pratique et facile à utiliser l'attelle peut en outre être proposée à plat, sous forme d'un kit, munie de sangles pré-positionnées.

Ainsi il est proposé selon l'invention une attelle qui est caractérisée en ce qu'elle est pourvue d'au moins un moyen de sanglage ajustable agencé autour de la circonférence du canal.

Ledit sanglage peut être obtenu par exemple au moyen de bandes présentant au moins une partie autocollante ou en velcro® ou tout autre moyen similaire connu de l'homme du métier permettant de maintenir solidaires les deux extrémités de la bande. Il peut bien entendu être envisagé selon l'invention de pratiquer des passants par exemple dans la semelle, les flancs ou les panneaux latéraux, pour maintenir la ou les bandes en place. Selon l'invention au moins une bande est positionnée pour maintenir l'attelle au niveau de la partie supérieure du membre immobilisé et au moins une seconde bande au niveau de sa partie inférieure. Le nombre de bandes variera en fonction de leur largeur. Si plusieurs bandes sont utilisées pour immobiliser une partie de membre elles sont idéalement disposées à intervalles réguliers.

De manière générale, selon l'invention il peut aussi être envisagé de pratiquer des entailles dans l'attelle permettant le passage de sangles ou des ouvertures destinées à favoriser la ventilation du membre. Enfin il peut être envisagé de pratiquer différentes fentes et languettes ou autres moyens de verrouillage connus de l'homme du métier pour s'assurer que l'attelle une fois pliée reste dans la forme que lui donne ce pliage sans qu'il soit besoin d'utiliser d'autres moyens. Bien entendu ce dernier mode de réalisation n'exclut pas que l'attelle soit par ailleurs maintenue par d'autres moyens tels que sangles, agrafes, points de collage et soudage par exemple si une matière plastique est utilisée.

Selon un autre aspect, l'invention a pour objet un kit pour réaliser une attelle telle que décrite plus haut, ledit kit se présentant sous une forme plate ou sensiblement plate après usinage, et pouvant être stocké à plat pour n'être plié, déplié ou assemblé sous la forme d'une attelle -s'il est composé de différentes pièces - qu'au moment de son utilisation.

Par souci de clarté, il sera fait référence à une « attelle » lorsque cette dernière est dépliée ou assemblée par opposition à un « kit » lorsque ladite attelle n'est pas encore pliée, dépliée ou assemblée, mais se présente sous une forme plate ou sensiblement plate.

Présenté à plat, le kit selon l'invention est composé d'au moins deux parties usinées dans la même feuille de carton ou matériau analogue. Sont délimitées par une ligne de pliage une première partie (« Partie I ») assurant la protection de la partie inférieure du membre et une seconde partie (« Partie II ») assurant la protection de la partie supérieure du membre. A ces deux parties peut être ajouté au moins un renfort, le tout taillé d'une pièce ou au moins un renfort taillé dans une pièce distincte.

Il est ainsi proposé un mode de réalisation de l'invention où l'attelle est présentée à plat dans une seule pièce, d'un seul tenant sous la forme d'un kit. Il s'agit d'un mode de réalisation préféré mais bien entendu l'invention recouvre également des modes alternatifs de réalisation par exemple où l'attelle est réalisée en plusieurs pièces distinctes.

Ainsi il est proposé selon l'invention un kit pour la réalisation d'une attelle telle que décrite plus haut.

Une telle description n'est pas limitative et l'attelle selon l'invention peut comporter une ou plusieurs autres parties destinées à améliorer son ergonomie. Il peut ainsi être envisagé d'intégrer aux parties I et II différents appendices, tels que notamment des languettes latérales formant sangles intégrées. Il peut aussi être pratiqué différentes découpes ou lignes de pliage supplémentaires dans le kit selon l'invention de manière à faciliter son pliage et/ou à le rendre plus ergonomique et/ou améliorer l'enveloppement du membre.

Ainsi selon l'invention il peut être pratiqué des lignes de pliage scindant les différentes parties du kit, telles que panneaux, flancs, semelle, tige, rabats etc... en différentes sous-parties.

Pour réduire l'espace nécessaire au stockage, le kit peut être plié sur lui-même, la partie I recouvrant la partie II.

L'attelle selon l'invention peut également être pourvue de moyens permettant l'immobilisation de la partie I par rapport à la partie II après pliage. Ces moyens peuvent par exemple être constitués d'éléments mâles disposés à l'extrémité des flancs et s'enclenchant dans des éléments femelles situés sur les panneaux latéraux ou à la jonction de ceux-ci avec la tige postérieure.

L'invention sera mieux comprise à la lecture des exemples suivants illustrés par les figures décrites ci-dessous (sur les figures les lignes discontinues représentent les lignes de pliage):
La figure 1 illustre un kit simple selon l'invention présenté à plat en vue du dessus composé d'une partie (I) destinée à protéger la partie inférieure du membre et d'une partie (II) destinée à protéger la partie supérieure du membre. Dans cette réalisation particulière la partie (I) présente deux extensions latérales (1.3) et (1.3') formant deux demi-sangles. La longueur de la partie (I) est inférieure à celle de la partie (II) cette attelle étant destinée à immobiliser un membre inférieur.

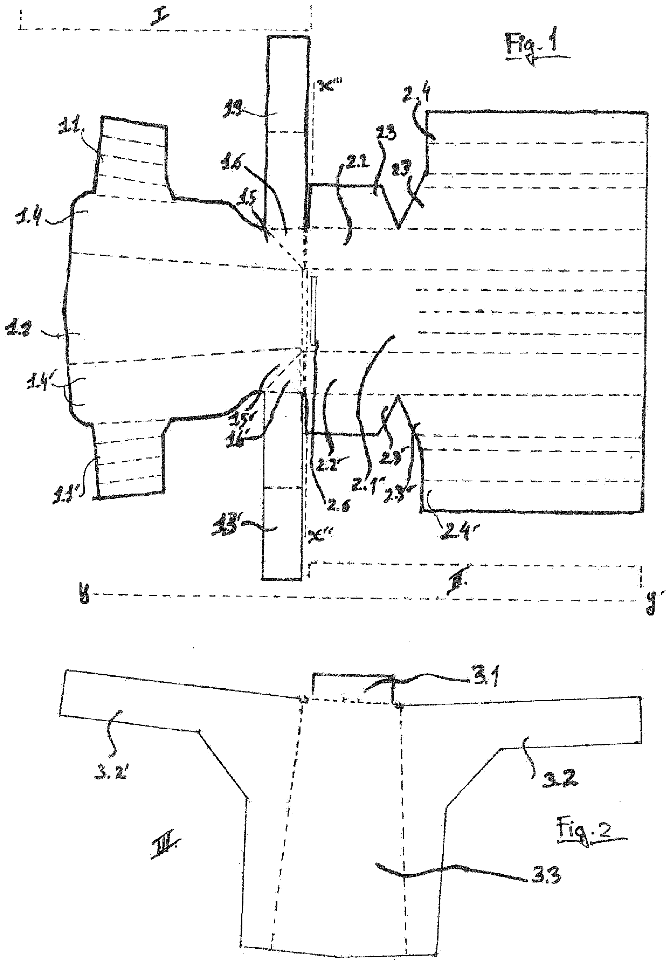

La figure 2 illustre une pièce de renfort selon l'invention présentée à plat en vue du dessus. Pour le montage, les deux équerres (3.2) et (3.2') sont érigées face à face en les pliant selon un angle de 90° par rapport à la partie centrale (3.3). Ce renfort peut alors être inséré sur la semelle (1.2) à l'intérieur de la partie (I) après érection des flancs (1.4) et (1.4') en vis-à-vis à 90° par rapport à la dite semelle (1.2), et érection de la partie (II) à 90° par rapport à la Partie (I), la languette de verrouillage (3.1) venant s'insérer dans la lumière de verrouillage (2.6) pratiquée à la base de la tige postérieure

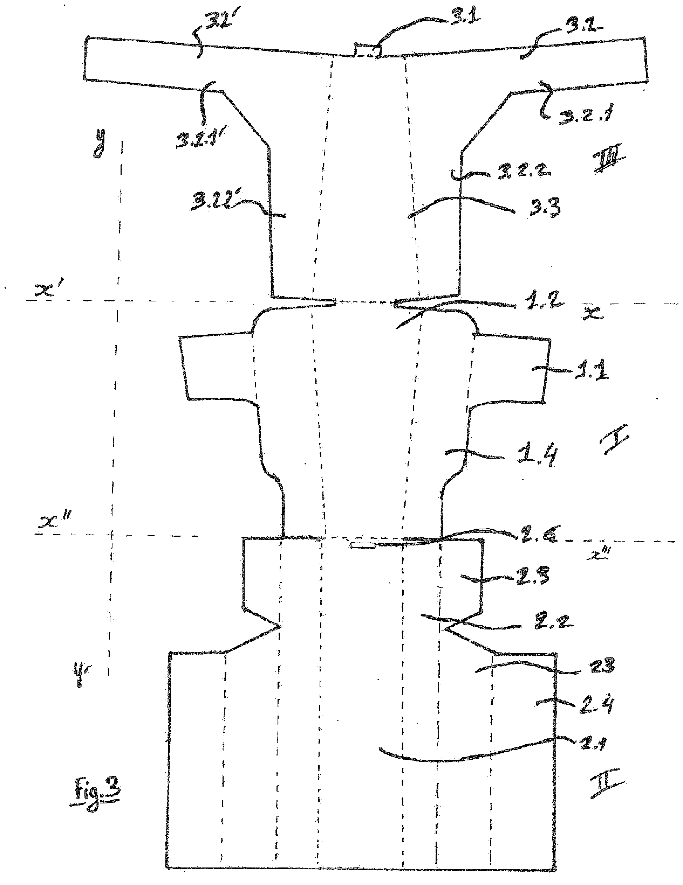

La figure 3 illustre un kit selon l'invention réalisé en une feuille présenté à plat en vue du dessus et comportant un renfort inférieur intérieur (III).

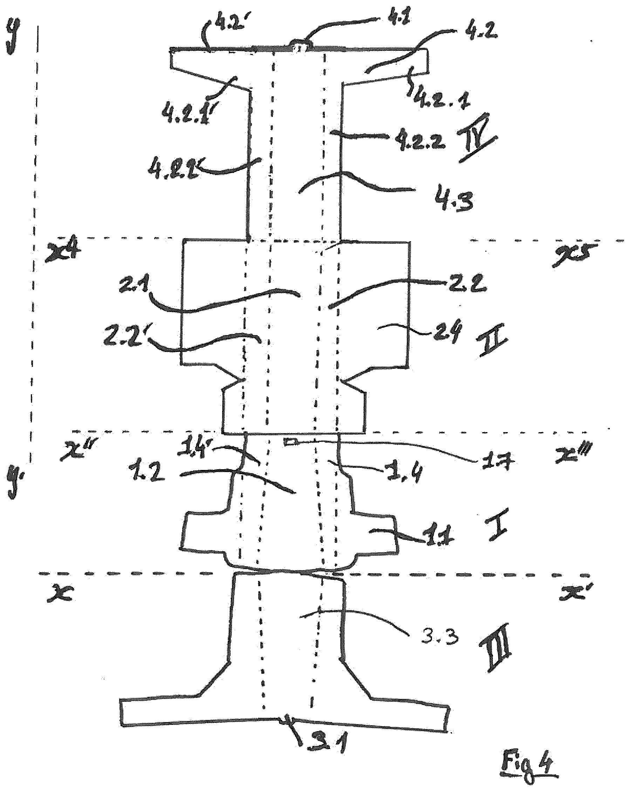

La figure 4 illustre un kit selon l'invention réalisé en une feuille présenté à plat en vue du dessus et comportant un renfort supérieur intérieur (VI) et un renfort inférieur intérieur (III).

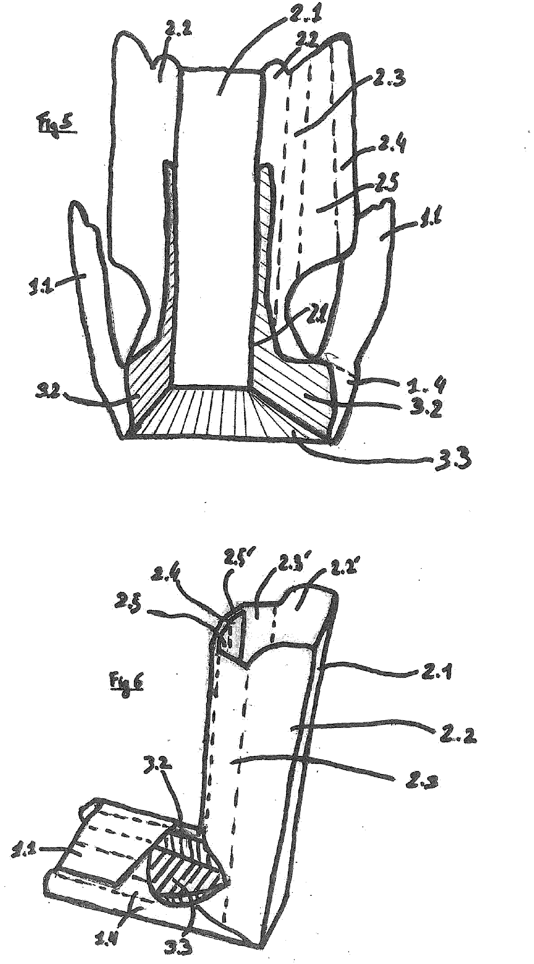

La figure 5 représente en vue de face une attelle pour jambe selon l'invention assemblée ouverte comportant un renfort inférieur intérieur (III) (partie hachurée).
La figure 6 représente une attelle pour jambe selon l'invention assemblée fermée en vue latéro-postérieure et comportant un renfort inférieur intérieur (III) (partie hachurée).

Bien entendu, les exemples ci-dessus ne sont rapportés qu'à titre d'illustration de l'invention, la portée de cette dernière étant définie par les revendications.

Toujours dans un souci de clarté, la nomenclature suivante s'applique aux figures illustrant l'invention :

### Partie I. Protection partie inférieure du membre.

(1.2) semelle
(1.4) et (1.4') flancs
(1.1) et (1.1') rabats
(1.5) et (1.5') triangles de jonctions
(1.6) et (1.6') triangles complémentaires de jonctions
(1.7) lumière de verrouillage

### Partie II. Protection partie supérieure du membre.

(2.1) tige postérieure
(2.2) et (2.2') panneaux latéraux
(2.3) et (2.3') panneaux intermédiaires
(2.4) et (2.4') panneaux antérieurs
(2.5) et (2.5') panneaux intermédiaires additionnels

### Partie III. Renfort intérieur de la partie inférieure du membre

(3.1) moyen de verrouillage
(3.2) et (3.2') équerres de renfort latéral
(3.3) partie centrale
(3.2.2) et (3.2.2') branches horizontales
(3.2.1) et (3.2.1') branches verticales

### Partie III'. Renfort extérieur de la partie inférieure du membre

(3.1) moyen de verrouillage
(3.2) et (3.2') équerres de renfort latéral
(3.3') partie centrale
(3.2.2) et (3.2.2') branches horizontales
(3.2.1) et (3.2.1') branches verticales

### Partie IV. Renfort intérieur de la partie supérieure du membre

(4.1) moyen de verrouillage
(4.3) partie centrale
(4.2) et (4.2') équerres de renfort latéral
(4.2.2) et (4.2.2') branches horizontales
(4.2.1) et (4.2.1') branches verticales

### Partie IV'. Renfort extérieur de la partie supérieure du membre

(4.1) moyen de verrouillage
(4.3') partie centrale
(4.2) et (4.2') équerres de renfort latéral
(4.2.2) et (4.2.2') branches horizontales
(4.2.1) et (4.2.1') branches verticales
Selon un mode de réalisation, l'invention a pour objet un kit caractérisé en ce qu'il comprend :
a) une partie (I) destinée à recevoir la partie inférieure du membre, ladite partie (I) comprenant i) une semelle (1.2), ii) des flancs (1.4 ; 1.4') s'étendant symétriquement de chaque côté de la semelle (1.2) sur tout ou portion de sa longueur et adaptés pour fléchir selon une ligne de pliage orientée suivant un axe longitudinal (y,y'), iii) au moins un rabat (1.1 ; 1.1') prolongeant latéralement le ou les flancs (1.4 ; 1.4') sur tout ou portion de leur longueur et adapté(s) pour fléchir selon une ligne de pliage orientée suivant un axe longitudinal (y, y'),
b) une partie (II) destinée à recevoir la partie supérieure du membre, prolongeant longitudinalement la partie (I) sur tout ou portion de sa largeur et adaptée pour fléchir (selon une ligne de pliage orientée suivant un axe transversal(x", x"') placé de manière adaptée pour être situé sensiblement à l'arrière de l'articulation, ladite partie (II) comprenant i) une tige postérieure (2.1) prolongeant longitudinalement la semelle (1.2) sur tout ou portion de sa largeur, ii) des panneaux latéraux (2.2 ; 2.2'), s'étendant latéralement de part et d'autre de la tige postérieure (2.1) sur tout ou portion de sa longueur et adaptés pour fléchir selon une ligne de pliage orientée suivant un axe longitudinal (y,y'), iii) des panneaux antérieurs (2.4 ; 2.4'), prolongeant latéralement les panneaux latéraux (2.2 ; 2.2') sur tout ou portion de leur longueur et adaptés pour fléchir selon une ligne de pliage orientée suivant un axe longitudinal (y, y')
c) au moins une partie de renfort latérale formant équerre sur tout ou partie des flancs latéraux (1.4 ; 1.4') et des panneaux latéraux (2.2 ; 2.2').

Selon un autre mode de réalisation des panneaux intermédiaires (2.3 ; 2.3'), prolongent latéralement les panneaux latéraux (2.2 ; 2.2') sur tout ou portion de leur longueur et sont adaptés pour fléchir selon une ligne de pliage orientée suivant un axe longitudinal (y, y') et des panneaux antérieurs (2.4 ; 2.4'), prolongent latéralement les panneaux intermédiaires (2.3 ; 2.3') sur tout ou portion de leur longueur et sont adaptés pour fléchir selon une ligne de pliage orientée suivant un axe longitudinal (y, y').

Selon une autre variante, afin de faciliter l'enveloppement des panneaux intermédiaires additionnels (2.5) et (2.5') peuvent être aménagés entre les panneaux antérieurs (2.4 ; 2.4') et les panneaux intermédiaires (2.3 ; 2.3').

Le passage de la forme plane (i.e. du kit) à la forme assemblée (i.e. l'attelle) s'effectue par :
a) pliage selon l'axe (x", x"') de la partie I et de la partie II selon un angle compris entre 135° et 45°.
b) pliage selon l'axe (y,y') des flancs (1.4) et (1.4')face à face et à 90° de la semelle (1.2),
c) pliage des panneaux latéraux (2.2) et (2.2'), l'un face à l'autre et à 90° par rapport à la tige postérieure (2.1),
d) le cas échéant immobilisation des parties I et II par l'enclenchement de moyens de verrouillage, par agrafage, collage ou tout autre moyen d'immobilisation similaire connu de l'homme du métier.
e) pliage des panneaux intermédiaires (2.3) et (2.3') l'un vers l'autre et respectivement selon un angle α compris entre 0° et 90° par rapport aux panneaux latéraux (2.2) et (2.2'), et
f) pliage des panneaux antérieurs (2.4) et (2.4') en recouvrement l'un de l'autre et selon un angle β égal à 90°-α par rapport aux panneaux intermédiaires respectivement (2.3) et (2.3').
g) pliage des rabats (1.1) et (1.1') en recouvrement l'un de l'autre et respectivement à 90° des flancs (1.4) et (1.4').

Selon l'invention l'attelle renforcée peut elle aussi être présentée à plat sous forme d'un kit. Le kit pour l'attelle renforcée selon l'invention peut comporter un ou deux renfort(s) placé(s) respectivement dans le prolongement de la partie I protégeant la partie inférieure du membre et/ou de la partie II protégeant la partie supérieure du membre.
Ainsi il est proposé selon l'invention un kit caractérisé en ce que au moins une partie de renfort latérale comprend au moins :
a) une partie de renfort inférieur choisie dans le groupe A comprenant :
   i)une partie de renfort inférieur intérieur (III) placée à l'opposé de la partie (II) en prolongement de la partie (I) et adaptée pour fléchir par rapport à cette dernière selon une ligne de pliage orientée suivant un axe transversal (x',x), ou
   ii) une partie de renfort inférieur extérieur (III') placée à l'opposé de la partie (II) en prolongement de la partie (I) et adaptée pour fléchir par rapport à cette dernière selon une ligne de pliage orientée selon un axe transversal (x',x)
      et/ou
b)une partie de renfort supérieure choisie dans le groupe B comprenant :
   i)une partie de renfort supérieur intérieur (IV) placée à l'opposé de la partie (I) en prolongement de la partie (II) et adaptée pour fléchir par rapport à cette dernière selon une ligne de pliage orientée selon un axe transversal (x4, x5) ou
   ii)une partie de renfort supérieur extérieur (IV') placée à l'opposé de la partie (I) en prolongement de la partie (II) et adaptée pour fléchir par rapport à cette dernière selon une ligne de pliage orientée selon un axe transversal (x4,x5).

Selon un mode de réalisation de l'invention le renfort inférieur intérieur (III) est adapté pour se replier à 180° et se loger à l'intérieur de la partie I une fois que ses différentes parties sont pliées pour former un canal. Selon un mode de réalisation de l'invention le renfort inférieur extérieur (III') est adapté pour se replier à 180° et insérer la partie I une fois que ses différentes parties sont pliées pour former un canal.

Selon un mode de réalisation de l'invention le renfort supérieur intérieur (IV) est adapté pour se replier à 180° et se loger à l'intérieur de la partie II une fois que ses différentes parties sont pliées pour former un canal. Selon un mode de réalisation de l'invention le renfort supérieur extérieur (IV') est adapté pour se replier à 180° et insérer la partie II une fois que ses différentes parties sont pliées pour former un canal.

Le passage de la forme plane (i.e. le kit) à la forme assemblée (i.e. l'attelle) ainsi renforcée s'effectue par
a) pliage selon l'axe (x",x"') de la partie I de la partie II selon un angle compris entre 45°et 135°,
b) pliage selon l'axe des (y, y') des flancs (1.4) et (1.4')face à face et à 90° de la semelle (1.2),
c) pliage des panneaux latéraux (2.2) et (2.2'), l'un face à l'autre et à 90° par rapport à la tige postérieure (2.1),
d) pliage des équerres de renfort latéral (3.2) et (3.2') à 90° par rapport à la partie centrale (3.3) ou le cas échéant (3.3'), en vis-à-vis l'une de l'autre et les branches verticales (3.2.1) et (3.2.1') dirigées à l'opposé de la tige postérieure (2.1)
d) le cas échéant immobilisation des parties I et II par l'enclenchement de moyens de verrouillage, par agrafage, collage ou tout autre moyen d'immobilisation similaire connu de l'homme du métier.

La mise en place du renfort inférieur intérieur (III) s'effectue par basculement à 180° selon l'axe (x,x') de la partie III ainsi formée de manière à ce qu'elle vienne se loger à l'intérieur du L formé à l'étape a), la partie centrale (3.3) en recouvrement de la semelle (1.2), les branches horizontales des équerres (3.2.2) et (3.2.2') en superposition à l'intérieur des flancs respectivement (1.4) et (1.4') et les branches verticales des équerres (3.2.1) et (3.2.1') en superposition à l'intérieur des panneaux latéraux respectivement(2.2)et (2.2').

Si l'attelle est pourvue d'un renfort inférieur extérieur (III') sa mise en place s'effectue par basculement à 180° selon l'axe (x,x') du renfort inférieur extérieur (III') ainsi formée de manière à ce qu'elle vienne insérer le L formé à l'étape a), la partie centrale (3.3') se plaçant sous la semelle (1.2), les branches horizontales des équerres (3.2.2) et (3.2.2') en superposition à l'extérieur des flancs respectivement(1.4) et (1.4') et les branches verticales des équerres (3.2.1) et (3.2.1') en superposition à l'extérieur des panneaux latéraux respectivement(2.2)et (2.2').

Si l'attelle est pourvue d'un renfort supérieur intérieur (IV) celui-ci se monte de manière similaire au renfort inférieur intérieur (III) ces deux renforts étant de conception générale identique.

Si l'attelle est pourvue d'un renfort supérieur extérieur (IV') celui-ci se monte de manière similaire au renfort inférieur extérieur (III') ces deux renforts étant de conception générale identique.
Selon une forme de réalisation, l'invention a pour objet un kit caractérisé en ce qu'il comprend au moins
a) une partie de renfort inférieur choisie dans le groupe A comprenant :
   i. une partie de renfort inférieur intérieur (III) placée à l'opposé de la partie II en prolongement de la partie (I) et adaptée pour fléchir par rapport à cette dernière selon une ligne de pliage orientée suivant un axe transversal (x',x), ladite partie de renfort inférieur intérieur (III) comprenant une partie centrale (3.3) ayant des dimensions identiques aux dimensions de la semelle (1.2), deux équerres de renfort latéral (3.2) et (3.2') dont la paire de premières branches horizontales (3.2.2) et (3.2.2') prolonge latéralement de part et d'autre la partie centrale (3.3) sur tout ou portion de sa longueur et adaptés pour fléchir par rapport à cette dernière selon une ligne de pliage orientée selon un axe longitudinal (y,y') et dont la seconde paire de branches verticales (3.2.1) et (3.2.1') est située à l'opposé de la ligne de pliage entre la partie (I) et la partie (II) ;
   ii. une partie de renfort inférieur extérieur (III') placée à l'opposé de la partie II en prolongement de la partie (I) et adaptée pour fléchir par rapport à cette dernière selon une ligne de pliage orientée selon un axe transversal (x',x), ladite partie de renfort inférieur extérieur (III') comprenant une partie centrale (3.3') ayant des dimensions égales aux dimensions de la semelle (1.2) augmentées latéralement de l'épaisseur de chacun des flancs (1.4) et (1.4'), deux équerres de renfort latéral (3.2)et (3.2') dont la paire de premières branches horizontales (3.2.2) et (3.2.2') prolonge latéralement de part et d'autre la partie centrale (3.3') sur tout ou portion de sa longueur et adaptés pour fléchir par rapport à cette dernière selon une ligne de pliage orientée selon un axe longitudinal (y,y') et dont la seconde paire de branches verticales (3.2.1) et (3.2.1') est située à l'opposé de la ligne de pliage entre la partie (I) et la partie (II)
      et/ou
b)une partie de renfort supérieur choisie dans le groupe B comprenant
   iii) une partie de renfort supérieur intérieur (IV) placée à l'opposé de la partie (I) en prolongement de la partie (II) et adaptée pour fléchir par rapport à cette dernière selon une ligne de pliage orientée selon un axe transversal (x4, x5) placée de manière adaptée pour être situé à l'extrémité de la tige postérieure (2.1) opposée à la partie (I), ladite partie de renfort supérieur intérieur (IV) comprenant une partie centrale (4.3) ayant des dimensions identiques aux dimensions de la tige postérieure (2.1), deux équerres de renfort latéral (4.2)et (4.2') dont la paire de premières branches horizontales (4.2.2) et (4.2.2') prolonge latéralement de part et d'autre la partie centrale (4.3) sur tout ou portion de sa longueur et adaptées pour fléchir par rapport à cette dernière selon une ligne de pliage orientée selon un axe longitudinal (y,y') et dont la seconde paire de branche verticales (4.2.1) et (4.2.1') est située à l'opposé de la ligne de pliage entre la partie (II) et la partie de renfort supérieur intérieur (IV).
   iv) une partie de renfort supérieur extérieur (IV') placée à l'opposé de la partie (I) en prolongement de la partie (II) et adaptée pour fléchir par rapport à cette dernière selon une ligne de pliage orientée selon un axe transversal (x4,x5) placée de manière adaptée pour être situé à l'extrémité de la tige postérieure (2.1) opposée à la partie (I), ladite partie de renfort supérieur extérieur (IV') comprenant une partie centrale (4.3') ayant des dimensions égales aux dimensions de la tige postérieure (2.1) augmentées latéralement de l'épaisseur de chacun des panneaux latéraux (2,2'), deux équerres de renfort latéral (4.2) et (4.2') dont la paire de premières branches horizontales (4.2.2) et (4.2.2') prolonge latéralement de part et d'autre la partie centrale (4.3') sur tout ou portion de sa longueur et adaptées pour fléchir par rapport à cette dernière selon une ligne de pliage orientée selon un axe longitudinal (y, y') et dont la seconde paire de branche verticales (4.2.1) et (4.2.1') est située à l'opposé de la ligne de pliage entre la partie (II) et la partie de renfort supérieur extérieur (IV').

La jonction entre la partie (I) et la partie (II), selon un angle compris entre 45° et 135° l'une par rapport à l'autre peut revêtir différentes formes selon l'invention.

Dans un premier mode de réalisation les extrémités des panneaux latéraux (2.2) et (2.2') situées au niveau de l'articulation viennent après pliage en superposition de l'extrémité des flancs (1.4) et (1.4'). Ils peuvent être assemblés par l'homme du métier à l'aide de tout moyen connu de lui, tel que colles, agrafes etc... Il peut aussi être envisagé parmi les nombreux modes de réalisation possibles de réduire l'extrémité des flancs (1.4) et (1.4') pour munir chacun d'une languette ou tout autre moyen de verrouillage décrit plus haut venant coopérer avec un autre moyen de verrouillage pratiqué à l'extrémité des panneaux latéraux (2.2) et (2.2'). Selon un autre mode de réalisation les flancs (1.4) et (1.4') ne sont pas réduits et le moyen de verrouillage de chaque panneau latéral coopère alors avec un moyen de verrouillage placé en vis-à-vis à la jonction des panneaux latéraux (2.2) et (2.2')et de la tige postérieure.

La jonction (1.5 ; 1.5') entre la partie I et la partie II de l'attelle selon l'invention peut également être élaborée sous forme de soufflet.

Bien entendu l'invention proposée recouvre également un soufflet similaire pratiqué sur les panneaux latéraux (2.2) et (2.2'). Pour un tel mode de réalisation il est avantageux pour faciliter le pliage de limiter la longueur des panneaux antérieurs et le cas échéant intermédiaires pour qu'ils ne s'étendent pas jusqu'à la zone où est aménagé le soufflet. Toujours selon un mode de réalisation de l'invention, il est décrit un kit caractérisé en ce qu'une ligne de pliage est pratiquée sur chaque flanc (1.4 ; 1.4') s'étendant en diagonale respectivement de la jonction entre la semelle (1.2), la tige (2.1) et chaque dit flanc (1.4) et (1.4') jusqu'à l'arrête opposée dudit flanc en formant une bissectrice à l'angle formé par la ligne délimitant la semelle (1.2) dudit flanc et la ligne de pliage (x", x"').
Au moment du pliage les triangles de jonctions (1.5) et (1.5') se placent en recouvrement des triangles complémentaires de jonctions (1.6) et (1.6'). Le pliage peut être complété par un point de colle, ou tout autre moyen de fixation équivalent, appliqué entre les triangles de jonctions (1.5) et (1.5') et les triangles complémentaires de jonctions (1.6) et (1.6').
Toujours selon un autre mode de réalisation de l'invention, il est décrit un kit caractérisé en ce qu'une ligne de pliage est pratiquée sur les panneaux latéraux (2.2) et (2.2') s'étendant en diagonale respectivement de la jonction entre la semelle (1.2), la tige (2.1) et chaque dit panneau latéral (2.2) et (2.2') jusqu'à l'arrête opposée dudit panneau latéral en formant une bissectrice à l'angle formé par la ligne délimitant la semelle (1.2) dudit panneau latéral et la ligne de pliage (x", x"').

## Revendications

1. Attelle enveloppante réalisée en feuille de carton ou matériaux analogues et constituée d'un profilé d'un seul tenant formant un angle compris entre 135° et 45° et comprenant de part et d'autre de cet angle:
a. une partie (I) d'immobilisation de la partie inférieure du membre comprenant :
i. une semelle (1.2),
ii. au moins deux flancs (1.4 ; 1.4') positionnés symétriquement le long de la semelle pour former un canal capable de recevoir la partie inférieure du membre, et
iii. au moins un rabat (1.1) agencé le long de l'un des flancs (1.4 ; 1.4') et adapté pour fléchir longitudinalement pour fermer par recouvrement ledit canal et envelopper la partie inférieure du membre ; et
b. une partie (II) d'immobilisation de la partie supérieure du membre comprenant :
i. au moins une tige postérieure (2.1),
ii. au moins deux panneaux latéraux (2.2 ; 2.2') positionnés symétriquement le long de la tige postérieure (2.1) pour former un canal capable de recevoir la partie supérieure du membre, et
iii. au moins un panneau antérieur (2.4) agencé le long de l'un des panneaux latéraux (2.2 ; 2.2') et adapté pour fléchir longitudinalement pour fermer par recouvrement ledit canal et envelopper la partie supérieure du membre
ladite attelle étant **caractérisée en ce que** ledit profilé coudé est doublé par au moins une partie de renfort latérale formant équerre au niveau de ce coude au moins sur tout ou partie des flancs latéraux (1.4 ; 1.4') et des panneaux latéraux (2.2 ; 2.2') de manière à assurer la rigidité dudit coude.

2. Attelle selon la revendication 1, **caractérisée en ce que** ladite au moins une partie de renfort latérale ledit doublage comprend au moins :
a. une partie de renfort inférieur choisie dans le groupe A comprenant :
i. une partie de renfort inférieur intérieur (III) ou
ii. une partie de renfort inférieur extérieur (III') et/ou;
b. une partie de renfort supérieur choisie dans le groupe B comprenant
i. une partie de renfort supérieur intérieur (IV) ou
ii. une partie de renfort supérieur extérieur (IV').

3. Attelle selon la revendication 2 **caractérisée en ce que** la partie de renfort inférieur choisie entre la partie de renfort inférieur intérieur (III) et la partie de renfort inférieur extérieur (III') comporte au moins un moyen de verrouillage adapté pour coopérer avec un moyen de verrouillage positionné sensiblement sur la ligne de pliage entre la partie (I) et la partie (II) ou sur la partie (II).

4. Attelle selon l'une des revendications 2 ou 3 **caractérisée en ce que** la partie de renfort supérieur choisie entre la partie de renfort supérieur intérieur (IV) et la partie de renfort supérieur extérieur (IV') comporte au moins un moyen de verrouillage adapté pour coopérer avec un moyen de verrouillage positionné sensiblement sur la ligne de pliage entre la partie (I) et la partie (II) ou sur la partie (I).

5. Attelle selon l'une quelconque des revendications précédentes, **caractérisée en ce qu'**elle comprend des languettes latérales (1.3) et (1.3') formant deux demi-sangles.

6. Attelle selon l'une quelconque des revendications précédentes **caractérisée en ce que** ses faces internes accueillant tout ou partie du membre sont garnies d'une ouate de protection.

7. Attelle selon l'une quelconque des revendications précédentes **caractérisée en ce qu'**elle est pourvue d'au moins un moyen de sanglage ajustable agencé autour de la circonférence du canal.

8. Kit pour la réalisation d'une attelle enveloppante réalisée en feuille de carton ou matériaux analogues **caractérisé en ce qu'**il comprend
a) une partie (I) destinée à recevoir la partie inférieure du membre, ladite partie (I) comprenant i) une semelle (1.2), ii) des flancs (1.4 ; 1.4') s'étendant symétriquement de chaque côté de la semelle (1.2) sur tout ou portion de sa longueur et adaptés pour fléchir selon une ligne de pliage orientée suivant un axe longitudinal (y,y'), iii) au moins un rabat (1.1 ; 1.1') prolongeant latéralement le ou les flancs (1.4 ; 1.4') sur tout ou portion de leur longueur et adapté(s) pour fléchir selon une ligne de pliage orientée suivant un axe longitudinal (y, y'),
b) une partie (II) destinée à recevoir la partie supérieure du membre, prolongeant longitudinalement la partie (I) sur tout ou portion de sa largeur et adaptée pour fléchir (selon une ligne de pliage orientée suivant un axe transversal (x", x"') placé de manière adaptée pour être situé sensiblement à l'arrière de l'articulation, ladite partie (II) comprenant i) une tige postérieure (2.1) prolongeant longitudinalement la semelle (1.2) sur tout ou portion de sa largeur, ii) des panneaux latéraux (2.2 ; 2.2'), s'étendant latéralement de part et d'autre de la tige postérieure (2.1) sur tout ou portion de sa longueur et adaptés pour fléchir selon une ligne de pliage orientée suivant un axe longitudinal (y,y'), iii) des panneaux antérieurs (2.4 ; 2.4'), prolongeant latéralement les panneaux latéraux (2.2 ; 2.2') sur tout ou portion de leur longueur et adaptés pour fléchir selon une ligne de pliage orientée suivant un axe longitudinal (y,y'),
c) au moins une partie de renfort latérale formant équerre sur tout ou partie des flancs latéraux (1.4 ; 1.4') et des panneaux latéraux (2.2 ; 2.2').

9. Kit selon la revendication 8, **caractérisé en ce que** ladite au moins une partie de renfort latérale comprend au moins :
a) une partie de renfort inférieur choisie dans le groupe A comprenant :
i. une partie de renfort inférieur intérieur (III) placée à l'opposé de la partie (II) en prolongement de la partie (I) et adaptée pour fléchir par rapport à cette dernière selon une ligne de pliage orientée suivant un axe transversal(x',x), ou
ii. une partie de renfort inférieur extérieur (III') placée à l'opposé de la partie (II) en prolongement de la partie (I) et adaptée pour fléchir par rapport à cette dernière selon une ligne de pliage orientée selon un axe transversal(x',x)
et/ou
b. une partie de renfort supérieur choisie dans le groupe B comprenant
i. une partie de renfort supérieur intérieur (IV) placée à l'opposé de la partie (I) en prolongement de la partie (II) et adaptée pour fléchir par rapport à cette dernière selon une ligne de pliage orientée selon un axe transversal (x4,x5) ou
ii. une partie de renfort supérieur extérieur (IV') placée à l'opposé de la partie (I) en prolongement de la partie (II) et adaptée pour fléchir par rapport à cette dernière selon une ligne de pliage orientée selon un axe transversal (x4,x5) .

10. Kit selon l'une quelconque des revendications 8 ou 9, **caractérisé en ce qu'**une ligne de pliage est pratiquée sur chaque flanc (1.4 ; 1.4') s'étendant en diagonale respectivement de la jonction entre la semelle (1.2), la tige (2.1) et chaque dit flanc (1.4) et (1.4') jusqu'à l'arrête opposée dudit flanc en formant une bissectrice à l'angle formé par la ligne délimitant la semelle (1.2) dudit flanc et la ligne de pliage (x", x"').

11. Kit selon l'une quelconque des revendications 8 ou 9 **caractérisé en ce qu'**une ligne de pliage est pratiquée sur les panneaux latéraux (2.2) et (2.2') s'étendant en diagonale respectivement de la jonction entre la semelle (1.2), la tige (2.1) et chaque dit panneau latéral (2.2) et (2.2') jusqu'à l'arrête opposée dudit panneau latéral en formant une bissectrice à l'angle formé par la ligne délimitant la semelle (1.2) dudit panneau latéral et la ligne de pliage (x", x"').

## Patentansprüche

1. Umwickelschiene, die aus einem Kartonblatt oder ähnlichen Materialien hergestellt ist und aus einem Profil aus einem einzigen Stück besteht, das einen Winkel zwischen 135° und 45° bildet und auf beiden Seitenwände dieses Winkels umfasst:
a . einen Teil (I) zur Immobilisierung des unteren Teils des Glieds, umfassend:
i. eine Sohle (1.2),
ii. mindestens zwei Seitenwände (1.4; 1.4'), die symmetrisch entlang der Sohle angeordnet sind, um einen Kanal zu bilden, der fähig ist, den unteren Teil des Glieds aufzunehmen, und
iii. mindestens eine Klappe (1.1), die entlang einer der Seitenwände (1.4; 1.4') angeordnet ist und dazu angepasst ist, sich in Längsrichtung zu biegen, um den Kanal durch eine Bedeckung zu schließen und den unteren Teil des Glieds zu umwickeln; und
b. einen Teil (II) zur Immobilisierung des oberen Teils ded Glieds, der umfasst:
i. mindestens eine hintere Stange (2.1),
ii. mindestens zwei seitliche Platten (2.2; 2.2'), die symmetrisch entlang der hinteren Stange (2.1) angeordnet sind, um einen Kanal zu bilden, der fähig ist, den oberen Teil des Glieds aufzunehmen, und
iii. mindestens eine vordere Platte (2.4), die entlang einer der seitlichen Platten (2.2; 2.2') angeordnet ist und dazu angepasst ist, sich in Längsrichtung zu biegen, um den Kanal durch eine Bedeckung zu schließen und den unteren Teil des Glieds zu umwickeln,
wobei die Schiene **dadurch gekennzeichnet ist, dass** das gebogene Profil durch mindestens einen seitlichen Verstärkungsteil verdoppelt ist, das einen Winkel bei der Biegung mindestens auf den gesamten seitlichen Seitenwände (1.4; 1.4') und den seitlichen Platten (2.2, 2.2') oder einem Teil davon bildet, um die Steifigkeit der Biegung zu gewährleisten.

2. Schiene nach Anspruch 1, **dadurch gekennzeichnet, dass** das mindestens eine seitliche Verstärkungsteil für die Verdopplung mindestens umfasst:
a. einen unteren Verstärkungsteil, der aus der Gruppe A ausgewählt ist, die umfasst:
i. einen inneren unteren Verstärkungsteil (III) oder
ii. einen äußeren unteren Verstärkungsteil (III') und / oder;
b. einen oberen Verstärkungsteil, der aus der Gruppe B ausgewählt ist, die umfasst:
i. einen oberen inneren Verstärkungsteil (IV) oder
ii. einen äußeren oberen Verstärkungsteil (IV').

3. Schiene nach Anspruch 2, **dadurch gekennzeichnet, dass** der untere Verstärkungsteil, der zwischen dem unteren inneren Verstärkungsteil (III) und dem unteren äußeren Verstärkungsteil (III') ausgewählt ist, mindestens ein Verriegelungsmittel aufweist, das dazu angepasst ist, mit einem Verriegelungsmittel zusammenzuwirken, das im Wesentlichen auf der Faltlinie zwischen dem Teil (I) und dem Teil (II) oder auf dem Teil (II) angeordnet ist.

4. Schiene nach einem der Ansprüche 2 oder 3, **dadurch gekennzeichnet, dass** der obere Verstärkungsteil, der zwischen dem oberen inneren Verstärkungsteil (IV) und dem oberen äußeren Verstärkungsteil (IV') ausgewählt ist, mindestens ein Verriegelungsmittel aufweist, das dazu angepasst ist mit einem Verriegelungsmittel zusammenzuwirken, das im Wesentlichen auf der Faltlinie zwischen dem Teil (I) und dem Teil (II) oder auf dem Teil (I) angeordnet ist.

5. Schiene nach einem der vorhergehenden Ansprüche, **dadurch gekennzeichnet, dass** sie seitliche Laschen (1.3) und (1.3') umfasst, die zwei Halbgurte bilden.

6. Schiene nach einem der vorhergehenden Ansprüche, **dadurch gekennzeichnet, dass** ihre Innenflächen, die das gesamte Glied oder einen Teil davon aufnehmen, mit einer Schutzwatte ausgekleidet sind.

7. Schiene nach einem der vorhergehenden Ansprüche, **dadurch gekennzeichnet, dass** sie mit mindestens einem einstellbaren Gurtmittel versehen ist, das um den Umfang des Kanals angeordnet ist.

8. Set zur Herstellung einer Umwickelschiene, die aus einem Kartonblatt oder ähnlichen Materialien hergestellt ist, **dadurch gekennzeichnet, dass** sie umfasst:
a) einen Teil (I), der dazu bestimmt ist, den unteren Teil des Glieds aufzunehmen, wobei der Teil (I) i) eine Sohle (1.2), (ii) Seitenwände (1.4; 1.4'), die sich symmetrisch an jeder Seite der Sohle (1.2) über die gesamte oder einen Abschnitt ihrer Länge erstrecken und dazu angepasst sind, sich entlang einer Faltlinie zu biegen, die entlang einer Längsachse (y, y') ausgerichtet ist, iii) mindestens eine Klappe (1.1; 1.1') umfasst, die die Seitenwand oder Seitenwände (1.4; 1.4') seitlich über ihre gesamte Länge oder einen Abschnitt ihrer Länge verlängert und dazu angepasst ist bzw. sind, sich entlang einer Faltlinie zu biegen, die entlang einer Längsachse (y, y') ausgerichtet ist,
b) einen Teil (II), der dazu bestimmt ist, den oberen Teil des Glieds aufzunehmen, der den Teil (I) in Längsrichtung über seine gesamte Breite oder einen Abschnitt davon verlängert und dazu angepasst ist, sich entlang einer Faltlinie zu biegen, die entlang einer Querachse (x", x"') ausgerichtet ist, die angepasst platziert ist, um im Wesentlichen an der Rückseite des Gelenks angeordnet zu sein, wobei der Teil (II) i) einen hinteren Schaft (2.1), der die Sohle (1.2) über ihre gesamte Breite oder einen Abschnitt davon verlängert, ii) seitliche Platten (2.2; 2.2'), die sich auf jeder Seite der hinteren Stange (2.1) über die gesamte Länge oder einen Abschnitt davon seitlich erstrecken und angepasst sind, sich entlang einer Faltlinie zu biegen, die entlang einer Längsachse (y, y') ausgerichtet ist, iii) vordere Platten (2.4, 2.4') umfasst, die die seitliche Platten (2.2; 2.2') seitlich über ihre gesamte Länge oder einen Abschnitt davon verlängern und angepasst sind, sich entlang einer Faltlinie zu biegen, die entlang einer Längsachse (y, y') ausgerichtet ist,
c) mindestens einen seitlichen Verstärkungsteil, der einen Winkel auf den gesamten seitlichen Seitenwänden (1.4; 1.4') und seitlichen Platten (2.2; 2.2') oder einem Teil davon bildet.

9. Set nach Anspruch 8, **dadurch gekennzeichnet, dass** der mindestens eine seitliche Verstärkungsteil mindestens umfasst:
a. einen unteren Verstärkungsteil, der aus der Gruppe A ausgewählt ist, die umfasst:
i. einen inneren unteren Verstärkungsteil (III), der gegenüber dem Teil (II) in Verlängerung des Teils (I) angeordnet ist und dazu angepasst ist, sich mit Bezug auf diesen letzteren entlang einer Faltlinie zu biegen, die entlang einer Querachse (x', x) ausgerichtet ist, oder
ii. einen äußeren unteren Verstärkungsteil (III'), der gegenüber dem Teil (II) in Verlängerung des Teils (I) angeordnet ist und dazu angepasst ist, sich mit Bezug auf diesen letzteren entlang einer Faltlinie zu biegen, die entlang einer Querachse (x', x) ausgerichtet ist,
und / oder
b. einen oberen Verstärkungsteil, der aus der Gruppe B ausgewählt ist, die umfasst:
i. einen oberen inneren Verstärkungsteil (IV), der gegenüber dem Teil (I) in Verlängerung des Teils (II) angeordnet ist und dazu angepasst ist, sich in Bezug auf diesen letzteren entlang einer Faltlinie zu biegen, die entlang einer Querachse (x4, x5) ausgerichtet ist, oder
ii. einen äußeren oberen Verstärkungsteil (IV'), der gegenüber dem Teil (I) in Verlängerung des Teils (II) angeordnet ist und dazu angepasst ist, sich in Bezug auf diesen letzteren entlang einer Faltlinie zu biegen, die entlang einer Querachse (x4, x5) ausgerichtet ist.

10. Set nach einem der Ansprüche 8 oder 9, **dadurch gekennzeichnet, dass** eine Faltlinie auf jeder Seitenwand (1.4; 1.4') ausgebildet ist, die sich jeweils diagonal von der Verbindung zwischen der Sohle (1.2), der Stange (2.1) und jeder Seitenwand (1.4) und (1.4') bis zu der gegenüberliegenden Kante der Seitenwand erstreckt, wobei sie eine Winkelhalbierende mit dem Winkel bildet, der durch die Linie gebildet ist, die die Sohle (1.2) der Seitenwand und die Faltlinie (x", x"') begrenzt.

11. Set nach einem der Ansprüche 8 oder 9, **dadurch gekennzeichnet, dass** eine Faltlinie an den seitlichen Platten (2.2) und (2.2') ausgebildet ist, die sich jeweils diagonal von der Verbindung zwischen der Sohle (1.2), der Stange (2.1) und jeder seitlichen Platte (2.2) und (2.2') bis zu der gegenüberliegenden Kante der seitlichen Platte erstreckt, wobei sie eine Winkelhalbierende mit dem Winkel bildet, der durch die Linie gebildet ist, der die Sohle (1.2) der seitlichen Platte und die Faltlinie (x", x"') begrenzt.

## Claims

1. Enveloping splint made from a sheet of cardboard or similar materials and consisting of a one-piece profile forming an angle between 135° and 45° and comprising on both sides of said angle:
a. a part (I) for immobilising the lower part of the limb comprising:
i. a sole (1.2),
ii. at least two sides (1.4; 1.4') positioned symmetrically along the sole to form a channel that is able to receive the lower part of the limb, and
iii. at least one flap (1.1) arranged along one of the sides (1.4; 1.4') and adapted to bend longitudinally in order to close by covering said channel and envelope the lower part of the limb; and
b. a part (II) for immobilising the upper part of the limb comprising:
i. at least one back element (2.1),
ii. at least two side panels (2.2; 2.2') positioned symmetrically along the back element (2.1) to form a channel that is able to receive the upper part of the limb, and
iii. at least one front panel (2.4) arranged along one of the side panels (2.2; 2.2') and adapted to bend longitudinally in order to close said channel by covering and envelope the upper part of the limb,
said splint being **characterised in that** said bent profile is overlapped by at least one side reinforcing part forming a bracket at said bend at least over all or part of the lateral sides (1.4; 1.4') and side panels (2.2; 2.2') so as to ensure the rigidity of said bend.

2. Splint according to claim 1, **characterised in that** said at least one side reinforcing part of said overlap comprises at least:
a. one lower reinforcing part selected from group A comprising:
i. a lower inner reinforcing part (III) or
ii. a lower outer reinforcing part (III') and/or;
b. an upper reinforcing part selected from group B comprising:
i. an upper inner reinforcing part (IV) or
ii. an upper outer reinforcing part (IV').

3. Splint according to claim 2, **characterised in that** the lower reinforcing part selected between the lower inner reinforcing part (III) and the lower outer reinforcing part (III') comprises at least one locking means adapted to cooperate with a locking means positioned substantially on the fold line between part (I) and part (II) or on part (II) .

4. Splint according to any of claims 2 or 3, **characterised in that** the upper reinforcing part selected between the upper inner reinforcing part (IV) and the upper outer reinforcing part (IV') comprises at least one locking means adapted to cooperate with a locking means positioned substantially on the fold line between part (I) and part (II) or on part (I).

5. Splint according to any of the preceding claims, **characterised in that** it comprises lateral flaps (1.3) and (1.3') forming two half-straps.

6. Splint according to any of the preceding claims, **characterised in that** its internal faces accommodating all or part of the limb are provided with protective wadding.

7. Splint according to any of the preceding claims, **characterised in that** it is provided with at least one adjustable strap arranged around the periphery of the channel.

8. Kit for making an enveloping splint made from a sheet of cardboard or similar materials, **characterised in that** it comprises,
a) a part (I) designed to receive the lower part of the limb, said part (I) comprising i) a sole (1.2), ii) sides (1.4; 1.4') extending symmetrically from each side of the sole (1.2) over all or a portion of its length and adapted to bend along a fold line oriented according to a longitudinal axis (y, y'), iii) at least one flap (1.1; 1.1') laterally extending the side or sides (1.4; 1.4') over all or a portion of their length and adapted to bend along a fold line oriented according to a longitudinal axis (y, y'),
b) a part (II) designed to receive the upper part of the limb, extending longitudinally part (I) over all or a portion of its width and adapted to bend according to a fold line oriented according to a transverse axis (x", x'") positioned in an adjusted manner so as to be located substantially behind the articulation, said part (II) comprising i) a back element (2.1) extending longitudinally the sole (1.2) over all or a portion of its width, ii) side panels (2.2; 2.2'), extending laterally on either side of the back element (2.1) over all or a portion of its length and adapted to bend along a fold line oriented according to a longitudinal axis (y, y'), iii) front panels (2.4; 2.4') laterally extending the side panels (2.2; 2.2') over all or a portion of their length and adapted to bend along a fold line oriented according to a longitudinal axis (y, y'),
c) at least one side reinforcing part forming a bracket over all or part of the lateral sides (1.4; 1.4') and the side panels (2.2; 2.2').

9. Kit according to claim 8, **characterised in that** said at least one side reinforcing part comprises at least:
a) a lower reinforcing part selected from group A comprising:
i. a lower inner reinforcing part (III) placed opposite part (II) extending the part (I) and adapted to bend relative to the latter along a fold line oriented according to a transverse axis (x', x), or
ii. a lower outer reinforcing part (III') placed opposite the part (II) extending from part (I) and adapted to bend relative to the latter along a fold line oriented according to a transverse axis (x', x)
and/or
b. an upper reinforcing part selected from group B comprising;
i. an upper inner reinforcing part (IV) placed opposite part (I) extending from part (II) and adapted to bend relative to the latter along a fold line oriented according to a transverse axis (x4, x5) or
ii. an upper outer reinforcing part (IV') placed opposite part (I) extending from part (II) and adapted to bend relative to the latter along a fold line oriented according to a transverse axis (x4, x5).

10. Kit according to any of claims 8 or 9, **characterised in that** a fold line is formed on each side (1.4; 1.4') extending diagonally respectively from the joint between the sole (1.2), the element (2.1) and each said side (1.4) and (1.4') up to the edge opposite said side by forming a bisector at the angle formed by the line delimiting the sole (1.2) of said side and the fold line (x", x'").

11. Kit according to any of claims 8 or 9, **characterised in that** a fold line is formed on the side panels (2.2) and (2.2') extending diagonally respectively from the joint between the sole (1.2), the element (2.1) and each said side panel (2.2) and (2.2') up to the opposite edge of said side panel by forming a bisector at the angle formed by the line delimiting the sole (1.2) of said side panel and the fold line (x", x'").
